# EUROPÄISCHE PATENTANMELDUNG

(11) **EP 4 624 542 A1**
(43) Veröffentlichungstag der Anmeldung: **01.10.2025**
(21) Anmeldenummer: 24167600.6
(22) Anmeldetag: 28.03.2024
(51) Int. Cl.: C09J 7/22, C08J 7/04, C09J 7/40

(54) **FOLIENANORDNUNG INSBESONDERE EINSCHLAGFOLIE FÜR HYGIENEPRODUKTE**

(71) Anmelder: Nitto Advanced Film Gronau GmbH, 48599 Gronau (DE); Nitto Denko Corporation, Ibaraki-shi, Osaka 567-8680 (JP)
(72) Erfinder: Homölle, Dieter, 48607 Ochtrup (DE)
(74) Vertreter: Andrejewski - Honke Patent- und Rechtsanwälte Partnerschaft mbB

(57) **Zusammenfassung**

Folienanordnung mit einer Trägerfolie (4) und einer auf der Trägerfolie (4) angeordneten Releasebeschichtung (6), wobei ein Hauptbestandteil der Trägerfolie (4) aus zumindest einem biologisch abbaubaren Polymer gebildet ist. Erfindungsgemäß ist zwischen der Trägerfolie (4) und der Releasebeschichtung (6) eine Barriereschicht (7) angeordnet.

## Beschreibung

Die vorliegende Erfindung betrifft eine Folienanordnung mit einer Trägerfolie und eine auf der Trägerfolie angeordneten Releasebeschichtung, wobei ein Hauptbestandteil der Trägerfolie aus zumindest einem biologisch abbaubaren Polymer gebildet ist.

Derartige Folienanordnungen sind aus der Praxis bekannt und kommen häufig als sogenannte Trennfolien zum Schutz von Oberflächen zum Einsatz und werden üblicherweise zur Abdeckung von Klebebändern oder anderweitigen klebrigen Oberflächen verwendet, um einerseits ein Verkleben während der Lagerung zu verhindern und, um zugleich auch die klebrige Oberfläche gegenüber Verschmutzungen zu schützen, sodass die Haftwirkung über einen möglichst langen Zeitraum aufrecht erhalten werden kann. Über die Releasebeschichtung wird eine möglichst rückstandsfreie Ablösung der darauf anhaftenden klebrigen Oberfläche ermöglicht. Zugleich wird über diese Releasebeschichtung aber auch eine vordefinierte Trennkraft festgelegt, sodass ein vorzeitiges Ablösen der darauf anhaftenden Oberfläche vermieden wird.

Derartige Folienanordnungen werden üblicherweise auch als Einschlagfolien für Hygieneprodukte, insbesondere für Damenbinden, verwendet. Hierbei wird dann das entsprechende Hygieneprodukt über eine Klebstoffschicht auf der Releasebeschichtung der Folienanordnung angeordnet. Die einzelnen Abschnitte der Folienanordnung können dann umgefaltet und versiegelt werden, sodass das Hygieneprodukt innerhalb einer von der Folienanordnung gebildeten Einschlagverpackung angeordnet ist. Für die Verwendung des Hygieneproduktes wird dann die Einschlagverpackung geöffnet und das Hygieneprodukt durch Abziehen von der Folienanordnung getrennt. Hierbei löst sich die Klebstoffschicht mit dem Hygieneprodukt ab und kann dann anschließend auf der textilen Oberfläche eines Kleidungsgegenstandes angeordnet werden, wobei über die Klebstoffschicht ein Verrutschen verhindert wird.

Üblicherweise kommen als Material für die Trägerfolie hauptsächlich Kunststoffe oder Papier zum Einsatz, wobei es sich bei den Kunststoffen insbesondere um Polyolefine, Polyester oder Polyamide handelt. Trägerfolien aus Polymeren weisen hierbei gegenüber Papier eine wesentlich größere Flexibilität und Beständigkeit auf und bewirken auch eine deutlich geringere Geräuschentwicklung beim Gebrauch der Verpackung. Allerdings gibt es in der Praxis zunehmend ein Bestreben, derartige Folienanordnungen möglichst aus biologisch abbaubaren Polymeren herzustellen, um die Nachhaltigkeit der daraus gebildeten Verpackungen zu verbessern. Hierzu kann dann die Trägerfolie vollständig oder zumindest aber ein Hauptbestandteil der Trägerfolie aus einem biologisch abbaubaren Polymer gebildet sein. Unter einem Hauptbestandteil der Trägerfolie wird hierbei ein Anteil der Trägerfolie verstanden, welcher zumindest 50 Gew.-%, bevorzugt zumindest 60 Gew.-%, besonders bevorzugt zumindest 80 Gew.-%, des Anteils der Trägerfolie ausmacht. Selbstverständlich kann die Trägerfolie auch vollständig aus zumindest einem biologisch abbaubaren Polymer gebildet sein.

Unter einem biologisch abbaubaren Polymer wird gemäß DIN EN 13432 ein Polymer verstanden, welches unter Einwirkung von Mikroorganismen und/oder Enzymen zu Wasser, Kohlendioxid und Biomasse zersetzbar ist und wobei unter festgelegten Bedingung nach 6 Monaten ein biologischer Abbau zu mindestens 90 %, bevorzugt zumindest 95 % besonders bevorzugt zu mehr als 98 %, erreicht wird. Hierbei kann das biologisch abbaubare Polymer sowohl aus synthetischen als auch aus natürlichen Rohstoffen gebildet sein. Eine entsprechende Folienanordnung ist beispielsweise in der EP 2 480 411 B1 beschrieben.

In der Praxis hat sich allerdings gezeigt, dass gerade bei Trägerfolien, welche zu einem großen Anteil aus biologisch abbaubaren Polymeren gebildet sind, bereits nach kurzer Zeit Probleme hinsichtlich der Haftwirkung der darauf angeordneten Klebstoffschicht ergeben. Insbesondere die nach FINAT FTM 11 bestimmte Restklebekraft der Releasebeschichtung fällt bereits nach kurzer Zeitdauer signifikant ab. Dieser Test wurde entwickelt, um die Aushärtung der Releasebeschichtung bei Folienanordnungen zu quantifizieren. Hierbei wird ein Testklebeband zunächst in einem bestimmten Winkel und in einer bestimmten Geschwindigkeit von einer Standard-Testplatte abgezogen und anschließend unter definierten Bedingungen in Kontakt mit einem releasebeschichteten Material gebracht und der Test wiederholt. Für beide Fälle werden die zum Abziehen erforderlichen Trennkräfte ermittelt und sodann in ein Verhältnis zueinander gesetzt, wobei üblicherweise Werte von über 80 % als optimaler Wert für die Restklebekraft verstanden werden. Sofern diese Werte deutlich darunter liegen, führt dies dazu, dass eine auf der Releasebeschichtung angeordnete Klebstoffschicht bereits nach kurzer Zeit einen signifikanten Abfall in der Haftwirkung aufweist, wobei der Test üblicherweise Aufschluss darüber gibt, ob nicht-ausgehärtete Rückstände der Silikonbeschichtung in die Klebstoffschicht übergehen und hierdurch die Haftwirkung herabsetzen.

Vor diesem Hintergrund liegt der Erfindung die Aufgabe zugrunde, eine Folienanordnung anzugeben, welche auch nach längeren Lagerzeiten noch immer eine ausreichende Haftwirkung einer auf der Releasebeschichtung angeordneten Klebstoffschicht aufweist.

Gegenstand und Lösung dieser Aufgabe ist eine Folienanordnung gemäß Patentanspruch 1. Demnach ist erfindungsgemäß vorgesehen, dass zwischen der Trägerfolie und der Releasebeschichtung eine Barriereschicht angeordnet ist. Hierbei wird sich auf eine im Wesentlichen homogene Ausbildung der Folienanordnung bezogen, sodass entsprechend die Barriereschicht vollflächig zwischen der Releasebeschichtung und der Trägerfolie angeordnet ist und diese entsprechend auch vollständig voneinander trennt.

Hierbei geht die Erfindung von der Erkenntnis aus, dass der Abfall in der Restklebekraft nicht auf nicht-ausgehärtete Rückstände der Releasebeschichtung zurückzuführen ist. Vielmehr migrieren Bestandteile der biologisch abbaubaren Polymere oder Compounds durch die Silikonbeschichtung hindurch in die Klebstoffschicht hinein und führen dazu, dass die Haftwirkung der Klebstoffschicht herabgesetzt wird. Beispiele für solche migrierenden Bestandteile sind Glycerinderivate oder Fettsäureester, die häufig als Verarbeitungshilfsmittel eingesetzt werden. Durch Einbindung einer Barriereschicht kann diese Migration wirksam verhindert werden, sodass trotz Verwendung biologisch abbaubarer Polymere in der Trägerfolie die Haftwirkung der Releasebeschichtung über einen vergleichsweise langen Zeitraum aufrechterhalten werden kann.

Gemäß einer bevorzugten Weiterbildung der Erfindung ist die Barriereschicht auf Basis von zumindest einem Acrylat gebildet. Entsprechend handelt es sich bei dem Acrylat um einen Hauptbestandteil der Barriereschicht. Unter Acrylaten werden Polymere verstanden, welche auf Basis von Estern der Acrylsäure gebildet sind. Die Erfindung umfasst unter anderem extrudierbare Acrylate, vorzugsweise Copolymere aus Ethylen und Acrylaten wie Ethylen-ButylacrylatCopolymer (EBA), Ethylen-Ethylacrylat-Copolymer (EEA), Ethylen-Methacrylat- 10-Copolymer (EMA) oder Ethylenacrylsäure-Copolymer (EAA).

Bevorzugt handelt es sich bei der Barriereschicht jedoch um einen ausgehärteten Acrylatlack, insbesondere um einen ausgehärteten Zwei-Komponenten-Acrylatlack. Bei einem solchen Lack wird das Acrylat in Lösemitteln wie Ethylacetat oder Methylethylketon auf die Folie aufgetragen. Danach verdampft das Lösemittel, so dass als Feststoff das Acrylat und gegebenenfalls zusätzliche Härter als Schicht auf der Folie verbleibt und durch Reaktion aushärtet, wobei dann der Lack auf eine vorgefertigte Trägerfolie aufgebracht wird. Bei einem Zwei-Komponenten-Acrylatlack wird dem Lack als erster Komponente eine zweite Komponente zugemischt. Hierbei handelt es sich bevorzugt um einen Härter, insbesondere auf Basis von Isocyanaten. Besonders bevorzugt handelt es sich um einen Härter auf Basis eines aromatischen Isocyanats. Bei dem Acrylat handelt es sich bevorzugt um ein Polyacrylat.

Insbesondere beträgt das Gewichtsverhältnis zwischen dem Acrylat und dem Härter zwischen 3:1 und 7:1, insbesondere zwischen 4:1 und 6:1.

Die Barriereschicht weist bevorzugt ein Auftragsgewicht von 0,05 bis 0,2 g/m², insbesondere zwischen 0,08 und 1,2 g/m² auf, wobei sich im Falle eines Acrylatlacks auf den ausgehärteten und getrockneten Zustand bezogen wird.

Zum Aufbringen eines Acrylatlacks eignen sich grundsätzlich verschiedene Auftragsverfahren, wobei gemäß einer bevorzugten Ausgestaltung der Acrylatlack mittels einem Tiefdruckverfahren, insbesondere mittels einem indirekten Tiefdruckverfahren, aufgebracht ist. Unter einem indirekten Tiefdruckverfahren wird eine Drucktechnik bezeichnet, bei der die Übertragung des Acrylatlacks nicht direkt von der Druckform auf die Trägerfolie erfolgt. Vielmehr wird der Acrylatlack zunächst auf eine Transferrolle übertragen und von dort aus auf die Trägerfolie. Insofern unterscheidet sich das Verfahren von einem direkten Tiefdruck, bei dem der Acrylatlack direkt von der Druckform auf die Trägerfolie aufgebracht wird. Dadurch ist es möglich, sehr dünne und gleichmäßige Schichten aufzutragen. Alternativ sind auch andere Auftragsverfahren denkbar wie z.B. Flexodruck.

Gemäß einer bevorzugten Weiterbildung der Erfindung beträgt der Anteil der Barriereschicht bezogen auf die Gesamtmasse der Folienanordnung weniger als 1 Gew.-%, bevorzugt weniger als 0,8 Gew.-%. Hierbei ist zu beachten, dass gemäß DIN EN 13432 Folien als kompostierbar angesehen werden, bei denen der Anteil einzelner nicht-biologisch abbaubarer Komponenten weniger als 1 Gew.-% und der Gesamtanteil der nicht-biologisch abbaubaren Komponenten weniger als 5 Gew.-% beträgt. Sofern die Trägerfolie ausschließlich aus biologisch abbaubaren Komponenten bzw. Polymeren gebildet ist, kann der Anteil der Barriereschicht und der Releasebeschichtung jeweils bis zu 1 Gew.-% betragen. Sofern auch weitere nicht-biologisch abbaubare Komponenten vorgesehen sind, ist der Anteil der Barriereschicht entsprechend zu verringern. Hierbei hat sich allerdings gezeigt, dass bereits vergleichsweise dünne Barriereschichten ausreichend sind, um wirksam eine Migration von Bestandteilen der Trägerschicht durch die Releasebeschichtung hindurch zu verhindern. Entsprechend kann somit selbst unter Einbindung einer im Wesentlichen nicht biologisch-abbaubaren Barriereschicht dennoch weiterhin eine Folienanordnung hergestellt werden, welche gemäß DIN EN 13432 weiterhin kompostierbar ist.

Gemäß einer bevorzugten Ausgestaltung der Erfindung, enthält die Trägerfolie zumindest ein Polymer aus nachwachsenden Rohstoffen. Grundsätzlich können Polymere aus nachwachsenden Rohstoffen auch biologisch abbaubar sein, wobei aber nicht zwangsläufig jedes Polymer aus nachwachsenden Rohstoffen auch biologisch abbaubar ist. Gleichermaßen können auch biologisch abbaubare Polymere synthetisch hergestellt werden, sodass sich zwischen biologisch abbaubaren Polymeren und Polymeren aus nachwachsenden Rohstoffen zwar eine gewisse Schnittmenge ergibt, diese aber voneinander zu unterscheiden sind. Der Vorteil einer Einbindung von Polymeren aus nachwachsenden Rohstoffen ist allerdings darin zu sehen, dass die Nachhaltigkeit der Folienanordnung insgesamt weiter verbessert wird.

Im Rahmen der Erfindung hat es sich als besonders wirkungsvoll herausgestellt, wenn das biologisch abbaubare Polymer bzw. die biologisch abbaubaren Polymere ausgewählt sind aus der Gruppe Polylactid (PLA), Polybutylenadipat-Terephthalat (PBAT), Polyhydroxyalkanoate (PHA, PHB, PHBV, PHBH), thermoplastische Stärke oder Mischungen hieraus. Sofern zumindest ein biologisch abbaubares Polymer eine Mischung aus den zuvor genannten biologisch abbaubaren Polymeren ist, so ist eine Mischung von Polylactid und Polybutylenadipat-Terephthalat besonders bevorzugt.

Bei der Trägerfolie kann es sich sowohl um einen einschichtigen als auch um einen mehrschichtigen Folienverbund handeln. Dieser wird vorzugsweise mittels Extrusion bzw. Coextrusion im Blas- oder Castfolienverfahren hergestellt. Im Falle einer mehrschichtigen Trägerfolie sind vorzugsweise sämtliche Schichten mit einem Hauptbestandteil, bevorzugt vollständig, aus einem biologisch abbaubaren Polymer gebildet. Dies schließt allerdings nicht aus, dass grundsätzlich auch Additive in der Trägerfolie oder zumindest in einer Schicht der Trägerfolie angeordnet sein können, wobei dann der Anteil bis zu 30 Gew.-%, bevorzugt bis zu 20 Gew.-%, besonders bevorzugt bis zu 15 Gew.-%, bezogen auf die Trägerfolie betragen kann.

Als Additive kommen insbesondere Farbmittel, Füllstoffe, Gleit- oder Antiblockmittel in Betracht, die in der Regel in Form eines Masterbatches zugemischt werden. Somit dienen die Additive der Einfärbung oder der Veränderung anderer Eigenschaften der entsprechenden Schicht bzw. Folie.

Bevorzugt können die Additive Calciumcarbonatpartikel (CaCO₃) enthalten oder aus diesen gebildet sein. Der Anteil von Calciumcarbonatpartikel in der Trägerfolie beträgt dann bevorzugt zwischen 10 und 35 Gew.-%, insbesondere zwischen 15 und 25 Gew.-%. Die Einbindung von Calciumcarbonatpartikeln hat hierbei den Vorteil, dass die noch ohne Releasebeschichtung ausgebildete Trägerfolie nicht ohne weiteres im aufgerollten Zustand gegeneinander verblockt. Zugleich wird auch das Verhalten der Trägerfolie während der Herstellung im Zuge der Extrusion wesentlich verbessert. Zugleich sind derartige anorganische Füllstoffe wie Calciumcarbonat gemäß der DIN EN 13432 bis zu einem Gehalt von 49% erlaubt, so dass die Folienanordnung grundsätzlich als kompostierbar bezeichnet werden kann, sofern die weiteren Voraussetzung hierfür erfüllt sind.

Gemäß einer Weiterbildung der Erfindung weisen die Calciumcarbonatpartikel eine Partikelgröße D50 zwischen 0,2 und 10 µm, insbesondere zwischen 0,5 und 5 µm, auf, was die Prozessfähigkeit und die Bioabbaubarkeit verbessern kann.

Wie bereits zuvor erläutert, dient die Releasebeschichtung dazu, eine darauf angeordnete Klebstoffschicht möglichst rückstandsfrei ablösen zu können. Dies kann beispielsweise dadurch bewirkt werden, dass die Releasebeschichtung zumindest eine silikonisierte Oberfläche aufweist. Besonders bevorzugt handelt es sich bei der Releasebeschichtung um eine im Wesentlichen vollständig aus Silikon gebildete Schicht. Unter einer im Wesentlichen vollständig aus Silikon gebildeten Schicht wird eine Schicht verstanden, bei der der Anteil des Silikons zumindest 95 Gew-% beträgt. Darüber hinaus können auch Restbestandteile z.B. Photoinitiatoren enthalten sein.

Das Silikon wird üblicherweise in einem flüssigen Zustand auf die Barriereschicht aufgebracht und härtet sodann aus. Hierbei kann das Silikon oder eine Silikonmischung zuvor erwärmt werden, um die Viskosität zu senken. Die Aushärtung kann hierbei entweder mittels Wärme (thermische Aushärtung) oder aber auch mittels UV-Strahlung erfolgen. Im Falle von UV-Strahlung kann es sich dann bei dem verwendeten Silikon um kationisch oder radikalisch aushärtende Silikone handeln, wobei gemäß einer bevorzugten Ausgestaltung der Erfindung die Releasebeschichtung aus einem radikalisch ausgehärteten Silikon gebildet ist. Eine radikalische Aushärtung weist hierbei den Vorteil auf, dass die Prozessdauer sehr stark verkürzt werden kann, da die Aushärtung schneller als bei einer kationischen Aushärtung und wesentlich schneller als bei einer reinen Wärmeaushärtung erfolgt. Bei temperatursensitiven Folien, insbesondere bei dünnen Folien aus Polymeren mit niedrigem Schmelzpunkt haben UV-härtende Systeme Vorteile gegenüber thermisch härtenden Systemen. Daher werden beispielsweise bei Polyethylenfolien in der Regel UVaushärtende Silikonsysteme, bei Polyesterfolien werden dagegen häufig thermisch-härtende Silikonsysteme eingesetzt.

Zur Aushärtung des Sillikons mittels UV-Strahlung wird insbesondere eine gewisse Menge an Photoinitiatoren als Restbestandteil dem Silikon beigemischt. Der Anteil in der Releasebeschichtung beträgt bis zu 5 Gew.-%, bevorzugt bis zu 3 Gew.-%.

Gemäß einer bevorzugten Weiterbildung der Erfindung beträgt das Auftragsgewicht der Releasebeschichtung zwischen 0,1 und 0,7, insbesondere zwischen 0,15 und 0,3 g/m², wobei sich insbesondere im Fall von Silikon auf den ausgehärteten Zustand bezogen wird.

Bevorzugt weist die Trägerfolie eine Dicke zwischen 15 und 40 µm, insbesondere zwischen 18 und 35 µm auf. Besonders bevorzugt beträgt die Dicke zwischen 20 und 32 µm.

Gemäß einer bevorzugten Weiterbildung der Erfindung ist die Folienanordnung gemäß DIN EN 13432 kompostierbar. Entsprechend weist nicht nur die Trägerfolie ein biologisch abbaubares Polymer auf. Vielmehr kann die ganze Folienanordnung kompostierbar sein. Gemäß der in Rede stehenden Norm kann eine Folienanordnung als kompostierbar bezeichnet werden, wenn die organischen Bestandteile mit einem Anteil von zumindest 95 %, insbesondere mit einem Anteil von zumindest 98 %, biologisch abbaubar bzw. kompostierbar sind.

Gemäß einer bevorzugten Weiterbildung der Erfindung liegt die Restklebkraft gemäß FINAT 11 unmittelbar nach der Produktion oberhalb von 80 %. Besonders bevorzugt liegt die Restklebkraft auch nach einer Lagerung von 2 Wochen bei 40 °C und 75 % relativer Luftfeuchte oberhalb von 80 %. Ganz besonders bevorzugt liegt die Restklebkraft nach einer Lagerung von 4 Wochen bei 40 °C und 75 % relativer Luftfeuchte oberhalb von 70 %.

Die Restklebkraft wird hierbei nach den Richtlinien von FINAT 11 (Stand 5. Edition, 1999) gemessen. Hierzu wird sowohl ein Testwert als auch ein Vergleichswert ermittelt, welche anschließend in ein Verhältnis zueinander gesetzt werden. In beiden Fällen wird zunächst eine Acetatfolie auf eine Breite von 3 cm und eine Länge von 12 cm zugeschnitten. Bei dieser Acetatfolie handelt es sich um eine Zellulose-Acetatfolie mit den Handelsnamen Tacphan AF 896 mit einer Dicke von 0,117 mm. Die Acetatfolie wird mit einem doppelseitigen Klebeband auf einem Blech fixiert und zur Bestimmung des Vergleichswertes anschließend ein Klebstoffstreifen mit einer Länge von 8 cm auf der Acetatfolie aufgebracht. Bei diesem Klebstoffstreifen handelt es sich um ein Klebeband des Typs Tesafilm 7475. Anschließend wird der Klebstoffstreifen mit einer FINAT Standardrolle (Gewicht 2050 g) zehnmal überrollt und sodann nach ca. 30 Sekunden unter einem Abzugswinkel von 180° und bei einer Abzugsgeschwindigkeit von 300 mm/min abgezogen. Hierbei wird dann die Klebkraft in N/Inch ermittelt.

Zur Bestimmung des Testwertes wird der Klebstoffstreifen zunächst auf der silikonisierten Folienseite der Folienanordnung aufgebracht und sodann zehnmal mit der Standardrolle überrollt. Sodann wird der Klebstoffstreifen nach ca. 60 Sekunden unter einem Abzugswinkel von 180° und bei einer Abzugsgeschwindigkeit von 300 mm/min abgezogen und anschließend auf der Acetatfolie aufgebracht. Sodann erfolgt das Vorgehen analog zur Bestimmung des Vergleichswertes. Insgesamt werden zur Versuchsdurchführung fünf Einzelbestimmungen pro Muster durchgeführt, und sodann der kleinste Testwert ermittelt. Der kleinste Testwert wird dann ins Verhältnis zum Vergleichswert gesetzt und hierdurch die Restklebekraft ermittelt.

Die Folienanordnung kann darüber hinaus auch eine Bedruckung aufweisen, wobei die Bedruckung insbesondere zwischen der Trägerfolie und der Barriereschicht angeordnet ist.

Gegenstand der Erfindung ist ferner eine Hygieneverpackung gemäß Anspruch 14. Insbesondere handelt es sich hierbei um eine Einschlagverpackung. Die Hygieneverpackung weist eine erfindungsgemäße Folienanordnung auf, wobei eine Klebstoffschicht zumindest abschnittsweise auf der Releasebeschichtung angeordnet ist. Bei dieser Klebstoffschicht handelt es sich insbesondere um einen Schmelzklebstoff (Hotmelt), welcher gemäß einer bevorzugten Weiterbildung einen Hygieneartikel, insbesondere eine Damenbinde, mit der Folienanordnung verbindet. Durch die Releasebeschichtung wird es ermöglicht, den Hygieneartikel zusammen mit der Klebstoffschicht rückstandsfrei zu entfernen, wobei durch die erfindungsgemäße Folienanordnung sichergestellt wird, dass die Klebstoffschicht auch nach einer längeren Lagerung noch immer eine ausreichend große Haftwirkung aufweist, da die Migration von Rückständen der Trägerfolie durch die Silikonschicht in die Klebstoffschicht hinein infolge der Einbindung der Barriereschicht verhindert wird. Entsprechend kommt gemäß einer solchen Ausgestaltung die Folienanordnung als Trennfolie zum Einsatz. Alternativ kann auch eine Klebebandanordnung vorgesehen sein, wobei dann anstelle eines Hygieneartikels ein Klebeband über die Klebstoffschicht an die Releasebeschichtung der Folienanordnung anschließt.

Gegenstand der Erfindung ist ferner ein Klebeband gemäß Anspruch 16 aus einer erfindungsgemäßen Folienanordnung, wobei eine Klebstoffschicht, insbesondere aus einem Schmelzklebstoff, auf einer der (ersten) Releasebeschichtung abgewandten Seite der Trägerfolie angeordnet ist. Mit der Releasebeschichtung wird somit insbesondere während der Herstellung verhindert, dass das Klebeband mit sich selbst verklebt wird.

Gemäß einer bevorzugten Ausgestaltung der Erfindung ist eine zweite Barriereschicht zwischen der Trägerfolie und der Klebstoffschicht angeordnet. Auch diese zweite Barriereschicht kann gemäß den vorstehenden Ausführungen ausgebildet sein, wobei über die beiden Barriereschichten sichergestellt wird, dass beispielsweise in einem aufgewickelten Zustand des Klebebandes keine Bestandteile der Trägerfolie in beide Richtungen in die Klebstoffschicht migrieren können und umgekehrt auch keine migrierfähigen Bestandteile der Klebstoffschicht in die Folie migrieren können, was beispielsweise verursachen kann, dass die Folie sich wellt.

Gemäß einer bevorzugten Weiterbildung der Erfindung weist die Folienanordnung eine zweite Releasebeschichtung zwischen der Klebstoffschicht und der zweiten Barriereschicht auf. Die zweite Releasebeschichtung kann hierbei insbesondere mit einer höheren Trennkraft gegenüber der Klebstoffschicht ausgebildet sein als die erste Releasebeschichtung.

Im Folgenden wird die Erfindung anhand von Ausführungsbeispielen näher erläutert. Die Figuren zeigen:
Fig. 1 eine Damenbinde auf einem Abschnitt einer erfindungsgemäßen Einschlagfolie im flachliegenden Zustand.
Fig. 2 eine einzeln in die Einschlagfolie eingeschlagene Damenbinde,
Fig. 3 einen Längsschnitt entlang der Linie A-A der Figur 1.

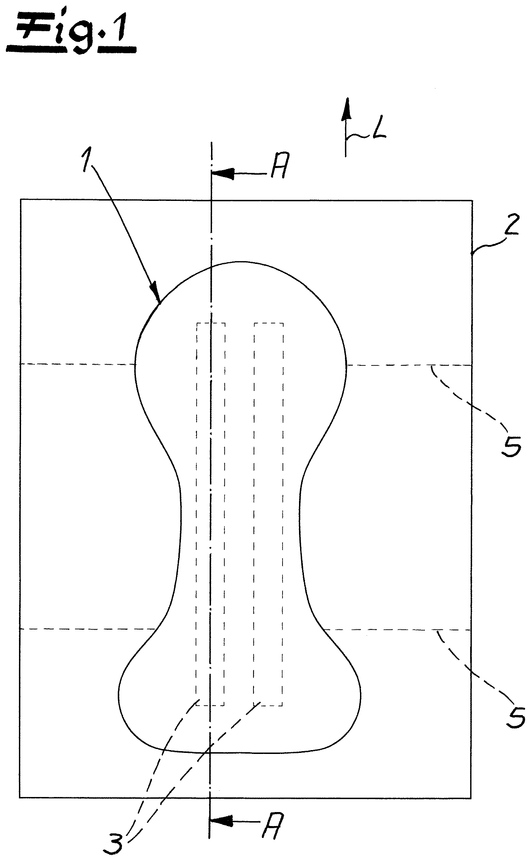

Die Figur 1 zeigt in einer Draufsicht in einem flachliegenden Zustand eine Damenbinde 1, welche auf einer als Einschlagfolie 2 aufliegt.

Um die Damenbinde 1 im Schnittbereich in einer Unterhose befestigen zu können, weist diese an ihre Unterseite Klebebereiche 3 aus einer Klebstoffschicht auf, wobei die Klebebereiche 3 der Figur 1 angedeutet sind. Die Klebebereiche 3 verlaufen als Streifen parallel zueinander entlang in eine Längsrichtung L der Damenbinde 1.

Die Damenbinde 1 kann in einem mittleren Abschnitt auch in nicht dargestellter Weise mit seitlichen Flügeln versehen sein, die zunächst nach oben geklappt sind und ebenfalls klebende Abschnitte aufweisen. Im Rahmen einer solchen in den Figuren nicht dargestellte Ausgestaltung ist dann eine separate Abdeckung der nach oben umgeklappten Flügel notwendig.

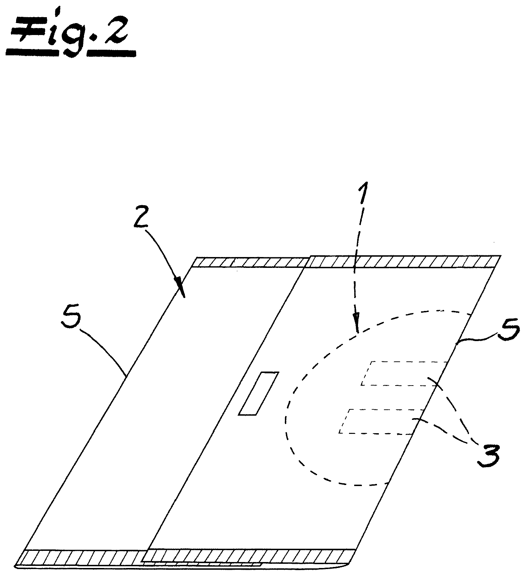

Die Figur 2 zeigt die mit der Einschlagfolie 2 einzeln verpackte Damenbinde 1, wobei die zunächst gemäß der Figur 1 flachliegenden Anordnung bezogen auf Längsrichtung L der Damenbinde 1 C-förmig zusammengefaltet ist, sodass dann in Längsrichtung L gesehen die Enden der Damenbinde 1 über ein Mittelabschnitt angeordnet sind. Aus der Figur 2 ersichtliche Knicklinien 5 sind zur Verdeutlichung auch in der Figur 1 eingezeichnet.

Die Einschlagfolie 2 wird aus einer Folienanordnung gebildet, welche dazu dient, die Damenbinde 1 einerseits zu schützen und um andererseits ein Abtrennen der Damenbinde 1 sowie der Klebstoffbereiche 3 zu ermöglichen. Hierbei ist es entscheidend, dass die Klebebereiche 3 bzw. die hierfür verwendeten Klebstoffschichten auch nach dem Abtrennen der Folienanordnung eine ausreichende Haftwirkung aufweisen, welche auch nach Lagerung über einen vergleichsweise langen Zeitraum gewährleistet wird.

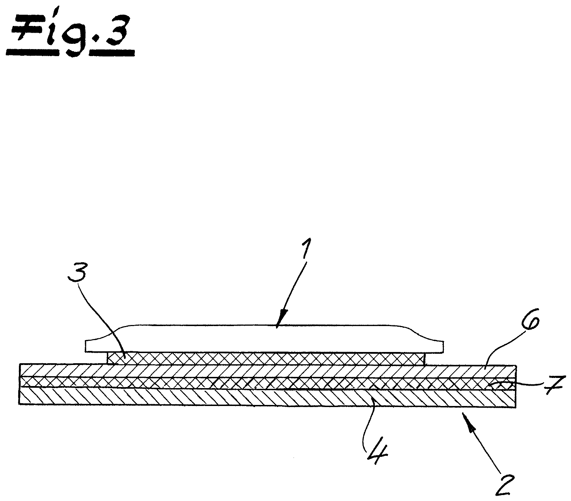

Die Figur 3 zeigt in diesem Zusammenhang einen Schnitt entlang der Linie A-A der Figur 1, wobei auch der Aufbau der für die Einschlagfolie 2 verwendeten Folienanordnung deutlich wird.

Gemäß üblicher Ausgestaltung weist die Folienanordnung eine Trägerfolie 4 sowie eine auf der Trägerfolie 4 angeordnete Releasebeschichtung 6 auf. Bei der Releasebeschichtung 6 handelt es sich üblicherweise um eine Silikonbeschichtung, welche im flüssigen Zustand aufgebracht wird und sodann aushärtet. Zur Aushärtung kommt insbesondere eine Aushärtung mittels UV-Strahlung in Betracht. Entsprechend handelt es sich bei der Releasebeschichtung 6 um eine radikalisch oder kationisch ausgehärtete Silikonschicht.

Mithilfe der Silikonschicht ist es möglich, einerseits eine vordefinierte Trennkraft zwischen den Klebebereichen 3 und der Folienanordnung einzustellen, wobei zugleich gewährleistet wird, dass die Klebebereiche 3 rückstandsfrei von der Folienanordnung entfernt werden können.

In der Vergangenheit wurden die Trägerfolien 4 üblicherweise aus synthetischen Polymeren wie z. B. Polyolefinen hergestellt, wobei aber zunehmend ein Bedarf besteht, die Trägerfolie 4 und damit auch die Folienanordnung insgesamt aus biologisch abbaubaren Polymeren herzustellen. Hierdurch soll insbesondere die Nachhaltigkeit derartiger Folienanordnungen verbessert werden, da es sich gerade im Hinblick auf die Verwendung als Einschlagfolie um Massenartikel handelt.

Hierbei zeigt sich allerdings, dass Rückstände der biologisch abbaubaren Polymere ausgehend von der Trägerfolie 4 durch die Releasebeschichtung 6 in die Klebebereiche 3 migrieren können und dort die Haftwirkung herabsetzen. Gerade nach verhältnismäßig kurzer Lagerdauer kann daher die Funktionalität der Klebebereiche 3 nicht mehr gewährleistet werden, sodass entsprechende Einschlagfolien 2 aus biologisch abbaubaren Polymeren von Nachteil sind.

Erfindungsgemäß ist nunmehr vorgesehen, dass zwischen der Releasebeschichtung 6 und der Trägerfolien 4 eine Barriereschicht 7 vorgesehen ist, welche die Trägerfolie 4 von der Releasebeschichtung 6 trennt und eine Migration von Rückständen aus der Trägerfolie 4 in die Releasebeschichtung 6 verhindert. Entsprechend können die Rückstände nicht in die Klebebereiche 3 gelangen, sodass die Haftwirkung der Klebebereiche 3 auch nach einen gewissen Lagerzeitraum gewährleistet werden kann.

Bei der Barriereschicht 7 handelt es sich insbesondere um eine Barriereschicht 7 auf Basis von zumindest einem Acrylat. Dieses Acrylat kann in Form eines Acrylatlacks, insbesondere eines Zwei-Komponenten-Acrylatlacks, auf die Trägerfolie 4 aufgebracht werden. Dies erfolgt beispielsweise mittels indirektem Tiefdruckverfahren. Im ausgehärteten Zustand erfolgt dann das Aufbringen der Releasebeschichtung 6.

Darüber hinaus kann die Folienanordnung auch eine Bedruckung vorsehen. Diese ist vorzugsweise auf einer der Releasebeschichtungen 6 abgewandten Seite der Trägerfolien 4 vorgesehen. Alternativ ist es aber auch möglich, eine Bedruckung zwischen der Releasebeschichtung 6 und der Trägerfolien 4 vorzusehen. Hierbei kann die Bedruckung entweder zwischen der Trägerfolie 4 und der Barriereschicht 7 oder aber auch zwischen der Barriereschicht 7 und der Releasebeschichtung 6 angeordnet sein.

### Ausführungsbeispiele

Für ein erstes Beispiel (B1) wurde eine erfindungsgemäße Folienanordnung mit einer Dicke von 25 µm hergestellt und zwischen der Trägerfolie 4 und der Releasebeschichtung 6 eine Barriereschicht 7 mit einem Auftragsgewicht von 0,1 g/m² aus einem 2K-Acrylatlack eingebunden. Zugleich wurden zwei Vergleichsbeispiele V1, V2 ohne eine entsprechende Barriereschicht 7 hergestellt, welche sich voneinander unterscheiden, indem für das Vergleichsbeispiel 1 (V1) ein spezielles Silikonsystem für biologisch abbaubare Polymere verwendet wurde. Der genaue Aufbau kann der Tab. 1 entnommen werden.

**Tabelle 1**

| | Beispiel 1 (B1) | | Vergleichsbeispiel 1 (V1) | | Vergleichsbeispiel 2 (V2) | |
|---|---|---|---|---|---|---|
| Trägerfolie | Dicke: 25 µm | | Dicke: 25 µm | | Dicke: 25 µm | |
| | Aufbau: | | Aufbau: | | Aufbau: | |
| | | 70 % PBAT-PLA-Ver. | | 70 % PBAT-PLA | | 70 % PBAT-PLA |
| | | 30 % Masterbatch | | 30 % Masterbatch | | 30 % Masterbatch |
| Barriereschicht | Auftragsgewicht: | | | | | |
| | 0,1 g/m² | | | | | |
| | Material: | | | | | |
| | 2K-Acrylatlack | | | | | |
| Releasebeschichtung | Auftragsgewicht: | | Auftragsgewicht: | | Auftragsgewicht: | |
| | | 0,2 g/m² | | 0,2 g/m² | | 0,2 g/m² |
| | Material: | | Material: | | Material: | |
| | Silikonsystem (Standard) | | Silikonsystem (Speziell für biol. abbaubare Polym.) | | Silikonsystem (Standard) | |

mit
PBAT-PLA-Verbindung:
   - ECOVIO F2332 (BASF)
   - PLA-Anteil: 10 Gew.-%
   - Dichte (ISO 1183): 1,25 g/cm³
   - Elastizitätsmodul MD (ISO 527): 150-500 Mpa
   - Elastizitätsmodul CD (ISO 527): 100-400 Mpa
   - Reißfestigkeit (DIN EN ISO 6383) MD: 500-1200 mN
   - Reißfestigkeit (DIN EN ISO 6383) CD: 300-900 mN
Masterbatch:
   - ECOVIO-basiert
   - Dichte (ISO 1183): 1,9 g/cm³
   - 60-70 Gew.-% CaCO₃-Partikel (Partikelgröße D50 = 0,5-5 µm)

Für das Beispiel 1 sowie die Vergleichsbeispiele 1 und 2 wurden sodann verschiedene Messungen durchgeführt, wobei die Art der Messungen sowie die Ergebnisse den nachfolgenden Tabellen 2 bis 4 entnommen werden können.

**Tabelle 2**

| | Lagerdauer | Glanz bei 45° [-] | Trennkraft [N/Inch] | Opazität [%] | Restklebkraft [%] |
|---|---|---|---|---|---|
| | | ISO 2813 | FINAT FTM 10 | DIN 6125 | FINAT FTM 11 |
| B1 | Frisch | 13,4 | 0,22 | 19,2 | 99,8 |
| | 2 Wochen (bei 40°C, 75% rel. LF) | 13,3 | 0,2 | 18,3 | 86,3 |
| | 3 Monate (bei 40°C, 75% rel. LF) | 14,0 | 0,24 | 20,1 | 68,4 |
| V1 | Frisch | 16,4 | 0,20 | 18,3 | 95,1 |
| | 2 Wochen (bei 40°C, 75% rel. LF) | 16,0 | 0,18 | 19,8 | 68,0 |
| | 3 Monate (bei 40°C, 75% rel. LF) | 15,5 | 0,18 | 20,4 | 59,8 |
| V2 | Frisch | 15,9 | 0,18 | 19,9 | 95,9 |
| | 2 Wochen (bei 40°C, 75% rel. LF) | 16,0 | 0,25 | 18,4 | 61,2 |
| | 3 Monate (bei 40°C, 75% rel. LF) | 14,7 | 0,28 | 19,2 | 60,4 |

**Tabelle 3**

| | Lagerdauer | Weiterreißfestigkeit MD [mN] | Weiterreißfestigkeit CD [mN] |
|---|---|---|---|
| | | DIN ISO 6383-2 | DIN ISO 6383-2 |
| B1 | Frisch | 3.413 | 1.169 |
| | 2 Wochen (bei 40°C, 75% rel. LF) | 3.688 | 923 |
| | 3 Monate (bei 40°C, 75% rel. LF) | 4.567 | 1.164 |
| V1 | Frisch | 3.860 | 1.365 |
| | 2 Wochen (bei 40°C, 75% rel. LF) | 3.772 | 1.093 |
| | 3 Monate (bei 40°C, 75% rel. LF) | 4.517 | 1.139 |
| V2 | Frisch | 2.785 | 1.303 |
| | 2 Wochen (bei 40°C, 75% rel. LF) | 3.412 | 921 |
| | 3 Monate (bei 40°C, 75% rel. LF) | 4.245 | 950 |

**Tabelle 4**

| | Lagerdauer | F-Max MD [N/Inch] | Dehnung-Max MD [%] | F-Max CD [N/Inch] | Dehnung-Max CD [%] |
|---|---|---|---|---|---|
| | | DIN EN ISO 527 | DIN EN ISO 527 | DIN EN ISO 527 | DIN EN ISO 527 |
| B1 | Frisch | 15,8 | 288 | 14,3 | 659 |
| | 2 Wochen (bei 40°C, 75% rel. LF) | 16,6 | 321 | 15,4 | 699 |
| | 3 Monate (bei 40°C, 75% rel. LF) | 13,4 | 273 | 11,7 | 423 |
| V1 | Frisch | 13,3 | 270 | 13,3 | 617 |
| | 2 Wochen (bei 40°C, 75% rel. LF) | 14,7 | 293 | 13,7 | 597 |
| | 3 Monate (bei 40°C, 75% rel. LF) | 14,4 | 294 | 14,4 | 293 |
| V2 | Frisch | 16,6 | 289 | 12,9 | 546 |
| | 2 Wochen (bei 40°C, 75% rel. LF) | 14,4 | 299 | 14,5 | 679 |
| | 3 Monate (bei 40°C, 75% rel. LF) | 12,5 | 274 | 10,3 | 510 |

Anhand der Tabellen ist ersichtlich, dass für jede Folienanordnung Messungen sowohl im frischen Zustand als auch nach einer gewissen Lagerdauer durchgeführt wurden. Unter einem frischen Zustand wird in diesem Zusammenhang der Zustand bezeichnen, welcher maximal bis 24 Stunden nach der Beschichtung mit der Releasebeschichtung 6 bei Raumtemperatur vorliegt. Entsprechend handelt sich um eine vergleichsweise kurze Zeitdauer, in der Migrationsvorgänge noch nicht im ausreichenden Maße erfolgen konnten.

Sodann wurde die Messung 2 Wochen nach Herstellung sowie 3 Monate nach der Herstellung erneut durchgeführt, wobei die Folienanordnung bei 40 °C und 75 % relativer Luftfeuchte gelagert wurde.

Anhand der Tab. 2 ist es ersichtlich, dass die Restklebekraft (gemessen nach FINAT FTM 11) nicht nur im frischen Zustand sondern insbesondere auch nach zweiwöchiger Lagerung deutlich höher ist als bei den Vergleichsbeispielen, welche ohne eine entsprechende Barriereschicht 7 hergestellt wurden.

Wenngleich nach einer dreimonatigen Lagerung nur noch Restklebkräfte unterhalb von 80° erreicht wurden, so lag der Wert mit 68,4 % weiterhin deutlich oberhalb der Werte die Vergleichsbeispiele, welche lediglich Werte im Bereich von max. 60 % erreichen konnten.

**Tabelle 5**

| | Beispiel 2 (B2) | Vergleichsbeispiel 3 (V3) | Vergleichsbeispiel 4 (V4) |
|---|---|---|---|
| Trägerfolie | Dicke: 25 µm | Dicke: 25 µm | Dicke: 25 µm |
| | Aufbau: | Aufbau: | Aufbau: |
| | 70 % PBAT-PLA | 90 % PBAT-PLA | 95 % PBAT-PLA |
| | 30 % Masterbatch | 10 % Masterbatch | 5 % Masterbatch |
| Barriereschic ht | Auftragsgewicht: | | |
| | 0,1 g/m² | | |
| | Material: | | |
| | 2K-Acrylatlack | | |
| | Härter | | |
| | Lack/Härter: 5:1 | | |
| Releasebeschichtung | Auftragsgewicht: | Auftragsgewicht: | Auftragsgewicht: |
| | 0,2 g/m² | 0,2 g/m² | 0,2 g/m² |
| | Material: | Material: | Material: |
| | Silikonsystem rad. aushärtend 2% Photoinititator | Silikonsystem rad. aushärtend 2% Photoinititator | Silikonsystem rad. aushärtend 2% Photoinititator |

**Tabelle 6**

| | Lagerdauer | Glanz bei 45° [-] | Trennkraft [N/Inch] | Restklebkraft [%] |
|---|---|---|---|---|
| | | ISO 2813 | FINAT FTM 10 | FINAT FTM 11 |
| B2 | Frisch | 11,9 | 0,23 | 91,0 |
| | 2 Wochen (bei 40°C, 75% rel. LF) | 11,9 | 0,20 | 77,3 |
| | 4 Wochen (bei 40°C, 75% rel. LF) | 13,2 | 0,21 | 73,1 |
| V3 | Frisch | 18,2 | 0,14 | 92,3 |
| | 2 Wochen (bei 40°C, 75% rel. LF) | 16,8 | 0,11 | 65,0 |
| | 4 Wochen (bei 40°C, 75% rel. LF) | 18,0 | 0,11 | 62,7 |
| V4 | Frisch | 17,1 | 0,14 | 90,8 |
| | 2 Wochen (bei 40°C, 75% rel. LF) | 18,8 | 0,11 | 57,4 |
| | 4 Wochen (bei 40°C, 75% rel. LF) | 19,3 | 0,10 | 57,4 |

**Tabelle 7**

| Beispiel 3 | Dicke | Zusammensetzung |
|---|---|---|
| Trägerfolie | 30 µm | 70 % PBAT-PLA-Ver. |
| | | 30 % Masterbatch |
| Barriereschicht | 0,1 g/m² | 2K-Acrylatlack |
| | | Lack/Härter: 5:1 |
| Releasebeschichtung | 0,2 g/m² | Silikonsystem rad. aushärtend |
| | | 2% Photoinititator |

**Tabelle 8**

| | | | |
|---|---|---|---|
| Glanz 45° | ISO 2813 | - | 12 |
| Trennkraft | FINAT FTM10 | N/Inch | 0,2 |
| Opazität | DIN 6125 | % | 25 |
| Restklebkraft | FINAT FTM 11 | % | 77 |
| Weiterreißfestigkeit MD | DIN ISO 6383-2 | mN | 5430 |
| Weiterreißfestigkeit CD | DIN ISO 6383-2 | mN | 1665 |
| COF (unbedruckte Seite gegen sich selbst) | ASTM D1894 | - | 0,51 |
| F-Max MD | DIN EN ISO 527 | N/Inch | 24,8 |
| F-Max CD | DIN EN ISO 527 | N/Inch | 23,4 |
| Dehnung Max MD | DIN EN ISO 527 | % | 252 |
| Dehnung Max CD | DIN EN ISO 527 | % | 719 |

Während die Herstellung der Folienanordnungen gemäß Tab. 1 zunächst im Labormaßstab erfolgte, wurden sodann Folienanordnungen im Produktionsmaßstab erzeugt, welchen in der Tab. 5 dargestellt sind. Das Beispiel 2 entspricht hierbei im Wesentlichen dem Beispiel 1. Als Releasebeschichtung 6 wurde ein Silikonsystem gewählt, welches radikalisch aushärtet und entsprechend 2 % eines Photoinitiators enthält. Als Barriereschicht 7 wurde erneut ein Zwei-Komponenten Acrylatlack verwendet, welcher eine Härter im Verhältnis 5:1 (Lack:Härter) enthält.

Die Vergleichsbeispiele 3 und 4 unterscheiden sich lediglich dahingehend voneinander, dass gemäß dem Vergleichsbeispiel 3 lediglich zu 10 % ein Masterbatch und gemäß dem Vergleichsbeispiel 4 5 % eines Masterbatches verwendet wurde. Sodann wurden erneuert die verschiedenen Folien zu unterschiedlichen Zeitpunkten vermessen, wobei diesmal eine Messung nach 2 Wochen sowie nach 4 Wochen erfolgte. Die Ergebnisse sind in der Tab. 6 aufgeführt. Auch hier ist deutlich zu erkennen, dass insbesondere die Restklebekraft für die erfindungsgemäße Folienanordnung gemäß dem Beispiel 2 deutlich oberhalb der Werte für die Vergleichsbeispiele liegt. Insbesondere wurden stets Werte oberhalb von 70 % erreicht.

Die Tab. 7 zeigt sodann eine weitere erfindungsgemäße Folienanordnung gemäß dem Beispiel 3. Der genaue Aufbau lässt sich der Tab. 7 entnehmen und zeigt, dass insbesondere eine nunmehr 30 µm dicke Trägerfolie 4 verwendet wurde. Die Ergebnisse nach einer Lagerung von 2 Wochen bei 40°C und 75% relativer Feuchte sind in der Tab. 8 aufgeführt, wobei insbesondere die Restklebekraft bei 77 % liegt.

## Patentansprüche

1. Folienanordnung mit einer Trägerfolie (4) und einer auf der Trägerfolie (4) angeordneten Releasebeschichtung (6), wobei ein Hauptbestandteil der Trägerfolie (4) aus zumindest einem biologisch abbaubaren Polymer gebildet ist.
**dadurch gekennzeichnet, dass**
zwischen der Trägerfolie (4) und der Releasebeschichtung (6) eine Barriereschicht (7) angeordnet ist.

2. Folienanordnung nach Anspruch 1, **dadurch gekennzeichnet, dass** die Barriereschicht (7) auf Basis von zumindest einem Acrylat gebildet ist.

3. Folienanordnung nach Anspruch 2, **dadurch gekennzeichnet, dass** die Barriereschicht (7) ein ausgehärteter Acrylatlack ist.

4. Folienanordnung nach Anspruch 3, **dadurch gekennzeichnet, dass** der ausgehärtete Acrylatlack ein ausgehärteter Zwei-Komponenten-Acrylatlack ist, welcher insbesondere einen Isocyanat-Härter enthält.

5. Folienanordnung nach einem der Ansprüche 1 bis 4, **dadurch gekennzeichnet, dass** der Anteil der Barriereschicht (7) bezogen auf die Gesamtmasse höchstens 1 Gew.-%, bevorzugt höchstens 0,8 Gew.-%, beträgt.

6. Folienanordnung nach einem der Ansprüche 1 bis 5, **dadurch gekennzeichnet, dass** die Trägerfolie (4) zumindest ein Polymer aus nachwachsenden Rohstoffen enthält.

7. Folienanordnung nach Anspruch 6, **dadurch gekennzeichnet, dass** zumindest ein biologisch abbaubares Polymer ein Polymer aus nachwachsenden Rohstoffen ist.

8. Folienanordnung nach einem der Ansprüche 1 bis 7, **dadurch gekennzeichnet, dass** das biologisch abbaubare Polymer ausgewählt ist aus der Gruppe Polylactid (PLA), Polybutylenadipat-Terephthalat (PBAT), Polyhydroxyalkanoat (PHA), Stärke oder Mischungen hieraus ist.

9. Folienanordnung nach einem der Ansprüche 1 bis 8, **dadurch gekennzeichnet, dass** die Trägerfolie (4) bis zu 25 Gew.-% Additive enthält.

10. Folienanordnung nach Anspruch 9, **dadurch gekennzeichnet, dass** die Additive zumindest teilweise aus Calciumcarbonatpartikeln (CaCO3) gebildet sind.

11. Folienanordnung nach Anspruch 10, **dadurch gekennzeichnet, dass** die Calciumcarbonatpartikel eine Partikelgröße D50 zwischen 0,2 und 10 µm, insbesondere zwischen 0,5 und 5 µm, aufweisen.

12. Folienanordnung nach einem der Ansprüche 1 bis 11, **dadurch gekennzeichnet, dass** die Releasebeschichtung (6) zumindest eine silikonisierte Oberfläche bildet oder aus Silikon gebildet ist.

13. Folienanordnung nach einem der Ansprüche 1 bis 12, **gekennzeichnet durch** eine Kompostierbarkeit gemäß DIN EN 13432.

14. Hygieneverpackung, insbesondere Einschlagverpackung, aus einer Folienanordnung nach einem der Ansprüche 1 bis 13, wobei eine Klebstoffschicht zumindest abschnittsweise auf der Releasebeschichtung (6) angeordnet ist.

15. Hygieneverpackung nach Anspruch 14, **dadurch gekennzeichnet, dass** ein Hygieneartikel, insbesondere eine Damenbinde (1), über die Klebstoffschicht mit der Folienanordnung verbunden ist.

16. Klebeband aus einer Folienanordnung nach einem der Ansprüche 1 bis 13, wobei eine Klebstoffschicht auf einer der Releasebeschichtung (6) abgewandten Seite der Trägerfolie (4) angeordnet ist.
